# EUROPEAN PATENT SPECIFICATION

(11) **EP 4 201 386 B1**
(45) Date of publication and mention of the grant of the patent: **21.01.2026**
(21) Application number: 21858060.3
(22) Date of filing: 06.07.2021
(51) Int. Cl.: A61F 13/535, A61F 13/494, A61F 13/511, A61F 13/534, A61F 13/537, A61F 13/539

(54) **ABSORBENT ARTICLE**
ABSORBIERENDER ARTIKEL
ARTICLE ABSORBANT

(30) Priority: 18.08.2020 JP 2020138218
(43) Date of publication of application: 28.06.2023
(73) Proprietor: Kao Corporation, Chuo-ku Tokyo 103-8210 (JP)
(72) Inventor: SUZUKI, Takeshi, Haga-gun, Tochigi 321-3497 (JP); TOMITA, Mina, Haga-gun, Tochigi 321-3497 (JP); SHIRAKAWA, Takashi, Haga-gun, Tochigi 321-3497 (JP)
(74) Representative: Vossius & Partner Patentanwälte Rechtsanwälte mbB
(86) International application number: PCT/JP2021/025457
(87) International publication number: WO 2022/038910

(56) References cited:
- JP-A- 2003 093 442
- JP-A- 2012 143 544
- JP-A- 2016 034 341
- JP-A- 2017 217 159
- JP-A- 2019 024 931
- JP-A- 2020 108 752

## Description

### Technical Field

The present invention relates to an absorbent article usable to absorb and retain excrements such as urine.

### Background Art

An absorbent article such as disposable diapers and sanitary napkins typically includes a topsheet to be placed relatively close to the skin of a wearer, a backsheet to be placed relatively distant from the skin of a wearer, and a liquid-retentive absorbent member between the sheets. As the absorbent member, a stack of a fiber material such as wood pulp or a member in which the stack supports absorbent polymer particles (hereinafter also collectively called a "stacked absorbent member") has been generally used. The stacked absorbent member is relatively bulky and thick and thus has excellent cushioning properties and the like, but gives an absorbent article having a poor or unattractive appearance due to its bulkiness. To address such disadvantages, as a thinner absorbent member than the stacked absorbent member, an absorbent member mainly including an absorbent polymer layer that is composed of substantially only an absorbent polymer and contains no fiber material (hereinafter also called a "sheet-type absorbent member") has been developed. As the sheet-type absorbent member, an absorbent member in which absorbent polymer particles are placed between two sheets placed to face together in the thickness direction is known.

US 2014/163504 A1 discloses a disposable diaper in which an acquisition-distribution system mainly containing a nonwoven fabric is placed between a topsheet and an absorbent core including a sheet-type absorbent member. The absorbent core extends longitudinally outward from the acquisition-distribution system, and the acquisition-distribution system has a larger basis weight in the front side than in the rear side. According to US 2014/163504 A1, the diaper effectively uses the absorbent core to prevent leakage.

JP 2008-237252 A discloses a disposable diaper including a stacked absorbent member having a two-layer structure. The skin-facing surface side of the diaper according to JP 2008-237252 A has leak-proof cuffs to rise toward a wearer wearing the diaper, and each longitudinal end portion of the leak-proof cuffs is fixed to other members in a portion overlapping with the corresponding longitudinal end portion of the absorbent member or with the vicinity thereof in plan view.

JP 2017 217159 A relates to an absorbent article having an absorbent body provided with an absorbent core and a pair of leakage prevention walls which are provided on both side parts in a width direction of the absorbent body and can rise on a skin side. The absorbent core has low basis weight parts lower in basis weight of the absorbent core than the surroundings.

### Summary of Invention

The invention is set out in the appended set of claims. Any references in the following description to embodiments, objects, aspects and/or examples which are not covered by the appended claims are considered as not being part of the present invention.

The present invention relates to an absorbent article that has a longitudinal direction corresponding to the front-rear direction of a wearer and a lateral direction orthogonal to the longitudinal direction, is sectioned into a crotch portion to be placed on the crotch section of a wearer, a front portion to be placed closer to the front side of the wearer than the crotch portion, and a rear portion to be placed closer to the rear side of the wearer than the crotch portion, and
includes an absorbent assembly and leak-proof cuffs placed at the respective sides along the longitudinal direction of the absorbent assembly, and the absorbent assembly includes an absorbent member to absorb and retain a body fluid and a topsheet placed on a skin-facing surface side of the absorbent member.

In an embodiment of the absorbent article of the present invention, each leak-proof cuff has, in the front portion and the rear portion, end portion fixing parts in which longitudinal end portions of the leak-proof cuff are fixed to the absorbent assembly, and a portion of the leak-proof cuff between the end portion fixing part in the front portion and the end portion fixing part in the rear portion is a stretch portion that is not fixed to other members and has elasticity in the longitudinal direction.

In an embodiment of the absorbent article of the present invention, the absorbent member includes a first layer and a second layer placed on a non-skin-facing surface side of the first layer and containing an absorbent polymer, and the second layer has, in at least one of the front portion and the rear portion, a longitudinal extension extending longitudinally outward from the first layer.

In an embodiment of the absorbent article of the present invention, the longitudinal extension of the second layer includes 1) a portion extending longitudinally outward from a first tangent that extends in the lateral direction, to a longitudinal end of the first layer or includes 2) when the first tangent is coincident with a second tangent that extends in the lateral direction, to a longitudinal end of the second layer, a portion defined by the coincident tangents and located at a nook portion of the absorbent member.

In an embodiment of the absorbent article of the present invention, in at least one of the front portion and the rear portion, the end portion fixing part overlaps with the longitudinal extension of the second layer in plan view, and the longitudinal end of the first layer is located inward longitudinally from the end portion fixing part and is located a space apart from the end portion fixing part.

In an embodiment of the absorbent article of the present invention, the longitudinal extension of the second layer has a smaller bending rigidity than the first layer and has a larger density of the absorbent polymer than the first layer.

Other features, effects, and embodiments of the present invention will be described below.

### Brief Description of Drawings

[Fig. 1] Fig. 1 is a developed plan view schematically showing a skin-facing surface side (topsheet side) of an open type disposable diaper in a spread out and stretched state as an embodiment of an absorbent article in the present invention.
[Fig. 2] Fig. 2 is a cross-sectional view schematically showing a cross section taken along line I-I in Fig. 1 (a cross section along the thickness direction of the crotch portion).
[Fig. 3] Fig. 3 is a plan view schematically showing a skin-facing surface side of a principal part of the absorbent member in Fig. 2.
[Fig. 4] Figs. 4 are sectional views schematically showing a section taken along line II-II in Fig. 1 (a lateral cross section along the thickness direction of an end portion fixing part of a leak-proof cuff in the rear portion and the vicinity thereof); Fig. 4(a) shows the diaper in an unworn state (in a spread out and stretched state); and Fig. 4(b) shows the diaper in a worn state (in a natural state).
[Fig. 5] Fig. 5 is a view schematically showing the diaper in Fig. 1 in a worn state.
[Fig. 6] Figs. 6 are views of still another embodiment of the absorbent article of the present invention, corresponding to Figs. 4; Fig. 6(a) shows a diaper in a spread out and stretched state (in an unworn state); and Fig. 6(b) shows the diaper in a worn state (in a natural state).
[Fig. 7] Fig. 7(a), Fig. 7(b), and Fig. 7(c) are plan views each schematically showing the positional relation between an absorbent member and end portion fixing parts of leak-proof cuffs in another embodiment of the absorbent article of the present invention.
[Fig. 8] Fig. 8(a) and Fig. 8(b) are views each showing an absorbent member in still another embodiment of the absorbent article of the present invention, corresponding to Fig. 3.
[Fig. 9] Fig. 9 is a view showing an absorbent member in still another embodiment of the absorbent article of the present invention, corresponding to Fig. 3.
[Fig. 10]Figs. 10 are views schematically showing a principal part of a conventional absorbent article (an open type disposable diaper); Fig. 10(a) is a view corresponding to Fig. 4(a); Fig. 10(b) is a view corresponding to Fig. 4(b); and Fig. 10(c) is a view corresponding to Fig. 5.

### Description of Embodiments

As described in JP 2008-237252 A, providing leak-proof cuffs on an absorbent article prevents excrements such as urine from flowing out in the lateral direction (what is called lateral leakage). However, excrements may leak not only in the lateral direction but also in the longitudinal direction (the direction corresponding to the front-rear direction of a wearer). Especially when a wearer wearing an absorbent article excretes excrements multiple times, excrements are highly probably leaked from the longitudinal end of the absorbent member to the outside. There is a demand for a technique capable of effectively preventing such leakage in the longitudinal direction.

The present invention therefore relates to an absorbent article having excellent leakage prevention performance in the longitudinal direction.

The present invention will now be described on the basis of preferred embodiments thereof with reference to drawings. In the following description of drawings, identical or similar components are indicated by an identical or similar sign. Drawings are basically schematic, and dimensional ratios and the like may differ from the actual ones.

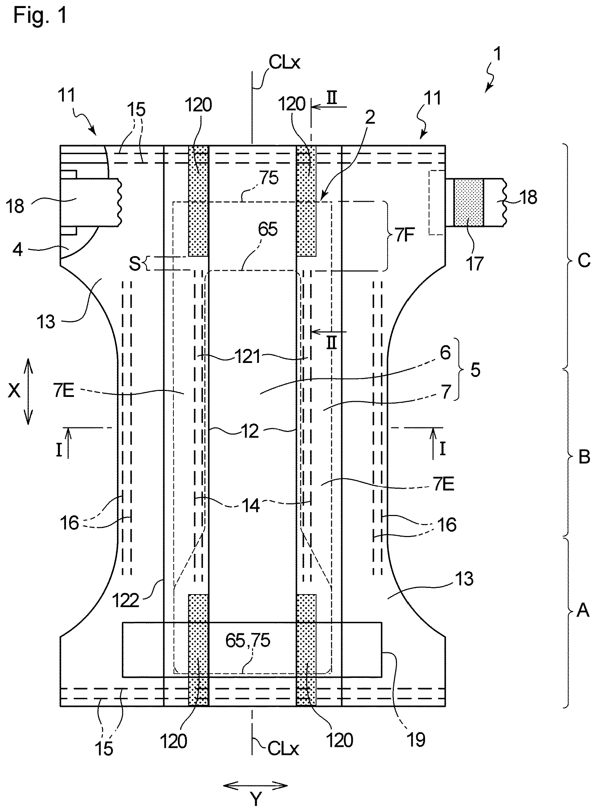

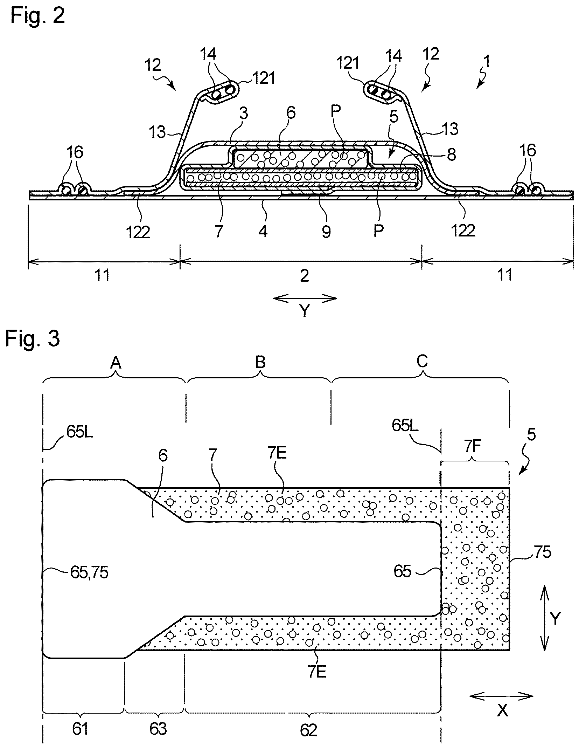

Fig. 1 and Fig. 2 show a disposable diaper 1 as an embodiment of the absorbent article of the present invention. The diaper 1 has a longitudinal direction X corresponding to the front-rear direction of a wearer, or to the direction extending from the front side through the crotch section to the rear side, and a lateral direction Y orthogonal to the longitudinal direction X. The diaper 1 is sectioned into three portions: a crotch portion B including an excretory-facing portion (not shown) to be placed on the crotch section of a wearer and to face the excretory including the penis; a front portion A to be placed closer to the front side (abdominal side) of a wearer than the crotch portion B; and a rear portion C to be placed closer to the rear side (dorsal side) of a wearer than the crotch portion B.

Each of the front portion A and the rear portion C typically includes a hip part to be placed on the below-waist of a wearer wearing the diaper 1. The front portion A is a part of the front body portion of the diaper 1, and the rear portion C is a part of the rear body portion of the diaper 1. The crotch portion B typically includes at least a part of the central portion in the longitudinal direction X of the diaper 1 and extends from the front body portion to the rear body portion of the diaper 1.

In the present invention, the front portion A, the crotch portion B, and the rear portion C may be trisected regions of a spread out and stretched diaper 1 in the longitudinal direction X. In the description, a "spread out and stretched state" is the state in which a diaper is spread out as shown in Fig. 1, and the spread out diaper is flattened out by stretching elastic members of each portion into design sizes (equal to the sizes of a flattened diaper where the effect of the elastic members is completely eliminated).

The diaper 1 includes an absorbent assembly 2 and leak-proof cuffs 12 placed at the respective sides along the longitudinal direction X of the absorbent assembly 2. The absorbent assembly 2 at least includes an absorbent member 5 to absorb and retain a body fluid and a topsheet 3 placed on a skin-facing surface side of the absorbent member and, in the present embodiment, further includes a backsheet 4 placed on a non-skin-facing surface side of the absorbent member 5.

The absorbent assembly 2 extends in the longitudinal direction X from the front portion A to the rear portion C, and the length direction thereof is coincident with the longitudinal direction X. A pair of the leak-proof cuffs 12 are placed in the central portion in the lateral direction Y of the diaper 1 and are spaced from each other. The topsheet 3, together with leak-proof sheets 13 included in the leak-proof cuffs 12, forms a skin-facing surface (inner surface) of the diaper 1, and the backsheet 4 forms a non-skin-facing surface (outer surface) of the diaper 1. The constituent members of the absorbent assembly 2 are joined together by a known joining means such as an adhesive. As the constituent members of the absorbent assembly 2, any member that has been typically used in this kind of absorbent article may be used provided that the following description for the corresponding member is applicable if any. As the topsheet 3, various liquid-permeable sheets may be used, and examples include a nonwoven fabric, a woven fabric, and paper. As the backsheet 4, a leak-proof sheet, specifically, a liquid-impermeable sheet (through which no liquid passes) or a sparingly liquid-permeable sheet (through which liquid is not completely prevented from passing but is difficult to pass) may be used, and examples include a moisture permeable resin film and a laminate of the resin film and a nonwoven fabric.

In the present description, a "skin-facing surface" is one face of an absorbent article or a constituent member thereof (for example, an absorbent member) and is the face facing the skin of a wearer at the time of wearing the absorbent article or is the face relatively close to the skin of a wearer, and a "non-skin-facing surface" is the other face of an absorbent article or a constituent member thereof and is the face facing opposite to the skin of a wearer at the time of wearing the absorbent article or is the face relatively distant from the skin of a wearer. In the present description, "at the time of wearing" means a condition in which a typical, appropriate wearing position or a normal wearing position of the absorbent article is maintained.

The diaper 1 includes flaps 11 extending outward from the peripheral edges of the absorbent assembly 2. The flap 11 includes members extending outward from the peripheral edges of the absorbent assembly 2 and is a portion without the absorbent member. In the present embodiment, as shown in Fig. 2, the topsheet 3 overlies the whole region of the skin-facing surface of the absorbent member 5, and the backsheet 4 overlies the whole region of the non-skin-facing surface of the absorbent member 5. The sheets 3 and 4 further extend laterally outward from the respective side edges along the longitudinal direction X of the absorbent member 5 and form, together with the leak-proof sheets 13, side flaps that are each a part of the flap 11. The side flap is a portion in the flap 11 and is located outside in the lateral direction Y from each side edge along the longitudinal direction X of the absorbent member 5 (the absorbent assembly 2) and from an imaginary extension line of the side edge. A plurality of members included in the flap 11 are joined together by a known joining means such as an adhesive, heat sealing, and ultrasonic sealing.

In the flaps 11 in the respective end portions (waist end portions) in the longitudinal direction X of the front portion A and the rear portion C, a plurality of waist gathering forming elastic members 15 are provided to be stretchable in the lateral direction Y, and these elastic members 15 are intermittently placed at certain intervals in the longitudinal direction X. The elastic members 15 are arranged while exhibiting the elasticity as described above, and thus in the waist end portions in the front portion A and the rear portion C where the elastic members are arranged, substantially continuous, annular waist gathers are formed over the entire circumference.

In each flap 11, in a leg portion to be placed around the leg of a wearer wearing the diaper 1, leg gathering forming elastic members 16 extensible in the longitudinal direction X extend in the longitudinal direction X over the entire length in the longitudinal direction X at least in the crotch portion B. At the time of wearing the diaper 1, shrinkage of the elastic members 16 forms leg gathers in the leg portion.

These gathering forming elastic members 15 and 16 in a stretched state are interposed between a plurality of sheets (in the present embodiment, two sheets of the topsheet 3, the backsheet 4, and the leak-proof sheet 13) included in the flap 11 and are fixed by a joining means such as an adhesive.

The above elastic members 14, 15 and 16 may be in any form, and filamentous members having, for example, a rectangular, regular square, circular, or polygonal cross section, string members (such as flat rubber), multifilament members, or the like may be used.

The diaper 1 is what is called an open type disposable diaper and includes, in each side edge portion along the longitudinal direction X in the rear portion C of the diaper 1, an attachment member 18 having an attachment portion 17 and, on the non-skin-facing surface of the backsheet 4 constituting the non-skin-facing surface of the front portion A, an attachment region 19 to which the attachment portions 17 are attachable, as shown in Fig. 1.

The leak-proof cuff 12 has, in the front portion A and the rear portion C, end portion fixing parts 120 in which end portions in the longitudinal direction X of the leak-proof cuff 12 are fixed to the absorbent assembly 2, and a portion of the leak-proof cuff 12 between the end portion fixing part 120 in the front portion A and the end portion fixing part 120 in the rear portion C is a stretch portion 121 that is not fixed to other members and has elasticity in the longitudinal direction X. The stretch portion 121 is present at least in the crotch portion B and may also be present in the front portion A and/or the rear portion C.

In the present embodiment, a pair of the leak-proof cuffs 12 are formed in the central portion in the lateral direction Y of the diaper 1 and are spaced from each other as shown in Fig. 1 and Fig. 2. The diaper 1 is formed symmetrically about a longitudinal center line CLx that bisects the diaper 1 in the lateral direction Y and virtually extends in the longitudinal direction X, and constituent members of the diaper 1 including the leak-proof cuffs 12 are formed symmetrically about the longitudinal center line CLx.

The leak-proof cuff 12 includes a liquid resistant or water repellent and breathable leak-proof sheet 13. The leak-proof sheet 13 is the main constituent of the leak-proof cuff 12 and extends in the longitudinal direction X over the entire length in the longitudinal direction X of the diaper 1 along the side edge along the longitudinal direction X of the absorbent assembly 2. Each end portion fixing part 120 of the leak-proof cuff 12 is a part in which the end portion in the longitudinal direction X of the leak-proof sheet 13 is fixed to the absorbent assembly 2 by a known fixing means including an adhesive such as a hot melt and heat sealing. As the leak-proof sheet 13, any member used as the material of the leak-proof cuff in this kind of absorbent article may be used, and for example, a single-layer or multi-layer water repellent nonwoven fabric or a laminate of a resin film and a nonwoven fabric may be used.

The leak-proof sheet 13 has an outer edge portion located at the outer side in lateral direction Y (the side relatively distant from the longitudinal center line CLx) and an inner edge portion located at the inner side in the lateral direction Y (the side relatively close to the longitudinal center line CLx). The outer edge portion of the leak-proof sheet 13 is fixed to other members (the topsheet 3 and the backsheet 4 in the embodiment in Fig. 2) by a known fixing means to form an edge portion fixing part 122. In the inner edge portion of the leak-proof sheet 13, each end portion in the longitudinal direction X is fixed to the absorbent assembly 2 to form an end portion fixing part 120 of the leak-proof cuff 12, and a portion between the end portions in the longitudinal direction X is not fixed to other members and is a free edge portion. The stretch portion 121 of the leak-proof cuff 12 is the free edge portion in the inner edge portion of the leak-proof sheet 13. To the leak-proof sheet 13 constituting the stretch portion 121, a single or multiple leak-proof cuff-forming elastic members 14 are fixed in a stretched state in the longitudinal direction X, and accordingly, the stretch portion 121 has elasticity in the longitudinal direction X. In the embodiment in Fig. 2, the inner edge portion of the leak-proof sheet 13 is folded in two in the lateral direction Y into a two-layer structure, and in the two-layer structure, two leak-proof cuff-forming elastic members 14 are fixed in a stretched state in the longitudinal direction X.

When the diaper 1 is worn, the shrinkage force of the leak-proof cuff-forming elastic members 14 allows the stretch portion 121 of each leak-proof cuff 12 between the pair of front and back end portion fixing parts 120 and 120 (the free edge portion of the leak-proof sheet 13) to rise from the edge portion fixing part 122 to other members of the leak-proof sheet 13 as a rising base end toward the skin of a wearer, and accordingly the leak-proof cuffs 12 rise to prevent lateral leakage. When the diaper 1 is worn, the shrinkage force of the leak-proof cuff-forming elastic members 14 pulls the front portion A and the rear portion C with the end portion fixing parts 120 toward the crotch portion B. Concurrently, the crotch portion B is curved to be convex on the non-skin-facing surface side (the side of the backsheet 4), and the whole diaper 1 is deformed into a boat shape. Such a boat-shaped diaper 1 easily fits the body shape of a wearer and has excellent wearing comfort and leakage prevention performance.

The absorbent member 5 includes a first layer and a second layer 7 that is placed on the non-skin-facing surface side of the first layer and contains an absorbent polymer P as shown in Fig. 2 and Fig. 3. In the diaper 1, a body fluid such as urine excreted by a wearer typically passes through a laminate structure of the first layer 6 and the second layer 7 in the order of the first layer 6 and the second layer 7. The second layer 7 has, in at least one of the front portion A and the rear portion C, a longitudinal extension extending longitudinally outward from the first layer 6.

In the present embodiment, the second layer 7 has the longitudinal extension 7F in the rear portion C but has no longitudinal extension 7F in the front portion A.

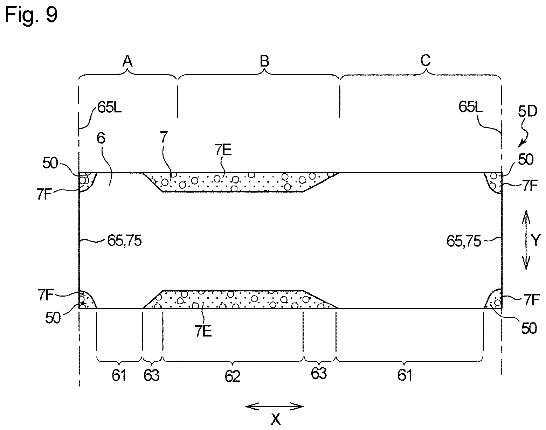

In the present invention, the longitudinal extension 7F of the second layer 7 includes the following portion 1) or 2). The absorbent member 5 in the embodiment includes the portion 1), whereas the absorbent member 5D described later includes the portion 2).
1) A portion extending longitudinally outward from a first tangent 65L that extends in the lateral direction Y, to a longitudinal end 65 of the first layer 6 or to the most distant point from the center in the longitudinal direction X of the first layer 6 on the side of the front portion A or the side of the rear portion C (see Figs. 3, 7, and 8).
2) When the first tangent 65L is coincident with a second tangent (not shown) extending in the lateral direction Y, to a longitudinal end 75 of the second layer 7, a portion defined by the coincident tangents and located at a nook portion 50 of the absorbent member (see Fig. 9).

In the present embodiment, the absorbent member 5 has a rectangular shape in plan view as shown in Fig. 3 and extends in the longitudinal direction X from the front portion A to the rear portion C, and the length direction thereof is coincident with the longitudinal direction X.

The second layer 7 has a rectangular shape in plan view and has a constant dimension (width) in the lateral direction Y, whereas the first layer 6 has varying widths, which vary in the longitudinal direction X. Specifically, as shown in Fig. 3, the first layer 6 has, in the front portion A, a maximum width portion 61 having the maximum dimension (width) in the lateral direction Y in the first layer 6, has, in the rear portion C, a minimum width portion 62 having the minimum width in the first layer 6, and has, between the maximum width portion 61 and the minimum width portion 62, a variable width portion 63 having widths that gradually increase or decrease from one side to the other side in the longitudinal direction X. In Fig. 3, the width of the variable width portion 63 gradually increases from the inside toward the outside in the longitudinal direction X (from the crotch portion B to the front portion A). The minimum width portion 62 is located at least in the crotch portion B and has a larger length in the longitudinal direction X than the maximum width portion 61 and the variable width portion 63. The first layer 6 has a smaller length in the longitudinal direction X than the second layer 7. On the side of the front portion A, the longitudinal ends 65 and 75 of the first layer 6 and the second layer 7 are coincident with each other, whereas the longitudinal end 65 of the first layer 6 on the side of the rear portion C is located inside in the longitudinal direction X (close to the crotch portion B) from the longitudinal end 75 of the second layer 7 on the side of the rear portion C.

In the present embodiment, as shown in Fig. 3, the second layer 7 extends outward from the peripheral edge of the first layer 6 in a portion other than the maximum width portion 61 (the minimum width portion 62 and the variable width portion 63), has the longitudinal extension 7F in the rear portion C, and has lateral extensions 7E extending laterally outward from the side edges along the longitudinal direction X of the first layer 6. The longitudinal extension 7F in the rear portion C and the lateral extensions 7E form an extension having a U shape in plan view.

In the present embodiment, as shown in Fig. 3, the side edges along the longitudinal direction X of the longitudinal end portion (maximum width portion 61) of the first layer 6 in the front portion A are located outside in the lateral direction Y from the side edges along the longitudinal direction X of the second layer 7 in a portion coincident in the longitudinal direction X with the longitudinal end portion (maximum width portion 61). In other words, in the longitudinal end portion in the front portion A, the first layer 6 protrudes in the lateral direction Y from the second layer 7. Such a structure improves the absorption capacity of the first layer 6, and consequently, the diaper 1 can have a higher liquid absorbability. A typical wearer of this kind of absorbent article (particularly, a disposable diaper) is likely to lie on the back while wearing the absorbent article, and thus it is important to improve the cushioning properties of the rear portion C as compared with the front portion A from the viewpoint of improving wearing comfort of the absorbent article. Hence, in the diaper 1, the longitudinal extension 7F of the second layer 7 is provided in the rear portion C to improve the cushioning properties of the rear portion C, whereas the first layer 6 in the front portion A has a larger width than the width of the second layer 7 to compensate a reduction in absorption capacity of the absorbent member 5 arising from the longitudinal extension 7F or a reduction in area of the first layer 6 in the rear portion C.

In the present embodiment, the absorbent member 5 includes a core-wrap sheet 9 overlying the outer surface of the laminate structure of the first layer 6 and the second layer 7, and the skin-facing surface and the non-skin-facing surface of the laminate structure is covered with the single core-wrap sheet 9. As the core-wrap sheet 9, a liquid permeable sheet may be used, and, for example, paper, a nonwoven fabric, or a woven fabric may be used. In the present invention, the absorbent member 5 may include no core-wrap sheet 9 or may include a plurality of core-wrap sheets 9. In the latter case, for example, the absorbent member 5 may include a single skin-facing core-wrap sheet overlying the skin-facing surface of the laminate structure of the first layer 6 and the second layer 7 and a single non-skin-facing core-wrap sheet overlying the non-skin-facing surface of the laminate structure.

In the present invention, each of the first layer 6 and the second layer 7 may be any member capable of absorbing and retaining a body fluid such as urine. Any member usable as the absorbent member (absorbent core) in this kind of absorbent article may be used, and the member may be either a stacked absorbent member or a sheet-type absorbent member. The first layer 6 and the second layer 7 may have the same structure or different structures provided that the second layer 7 (in the absorbent member, the layer relatively distant from the skin of a wearer wearing the absorbent article) contains an absorbent polymer. For example, both the first layer 6 and the second layer 7 may be a stacked absorbent member or a sheet-type absorbent member, or one of them may be a stacked absorbent member, and the other may be a sheet-type absorbent member. The first layer 6 may contain no absorbent polymer and, for example, may consist of a fiber aggregate of an absorbent fiber material such as wood pulp.

In the present embodiment, the first layer 6 is a stacked absorbent member, and the second layer 7 is a sheet-type absorbent member. The layers 6 and 7 will next be described.

The first layer 6 has a structure in which a stack of a fiber material supports an absorbent polymer. Sign P in the drawings is an absorbent polymer. The first layer 6 is typically composed of a mixed layer of the fiber material and the absorbent polymer.

In the first layer 6, the absorbent polymer may be uniformly distributed throughout the first layer 6 or may be unevenly distributed. The former is a typical distribution pattern. A specific example of the latter is a pattern in which an absorbent polymer is present mainly in the skin-facing surface side or the non-skin-facing surface side of the first layer 6 or a pattern in which the content of an absorbent polymer is larger in one of the skin-facing surface side and the non-skin-facing surface side than the other. In the description, the skin-facing surface side of a first layer 6 and the non-skin-facing surface side of the first layer 6 are defined by an imaginary straight line (not shown) that bisects the first layer 6 in the thickness direction.

As the fiber material included in the first layer 6, any material usable in this type of absorbent member may be used, and a single material or a mixture of two or more materials may be used. The fiber material is preferably an absorbent fiber material. Examples of the absorbent fiber material include natural fibers such as wood pulps including softwood pulp and hardwood pulp and non-wood pulps including cotton pulp and hemp pulp; modified pulps such as cationized pulp and mercerized pulp (i.e., cellulose fibers); and hydrophilic synthetic fibers.

As the absorbent polymer included in the first layer 6, any material usable in this type of absorbent member may be used, and typically a hydrogel material capable of absorbing and retaining water may be used. For example, a polymer or a copolymer of acrylic acid or an alkali metal acrylate may be used. Examples thereof include polyacrylic acid, salts thereof, polymethacrylic acid, and salts thereof and specifically include a partial sodium salt of acrylic polymer. The absorbent polymer may have any shape and may be, for example, spherical, tufted, massive, ellipsoid, fibrous, or indefinite particles or particles having a combination shape thereof.

The basis weight of the first layer 6, the content of a forming material, and the like are not specifically limited but are preferably set at the following values from the viewpoint of allowing the first layer 6 to certainly achieve intended effects.

The basis weight of the first layer 6 is preferably 10 g/m² or more and more preferably 50 g/m² or more and is preferably 600 g/m² or less and more preferably 500 g/m² or less.

The content of the fiber material in the first layer 6 is preferably 5% by mass or more and more preferably 10% by mass or more and is preferably 70% by mass or less and more preferably 65% by mass or less relative to the total mass of the first layer 6.

The content of the absorbent polymer in the first layer 6 is preferably 30% by mass or more and more preferably 35% by mass or more and is preferably 95% by mass or less and more preferably 90% by mass or less relative to the total mass of the first layer 6.

The first layer 6 may be produced by using a known fiber stacking apparatus with a rotating drum by a common method. The fiber stacking apparatus typically includes a rotating drum having, on the outer peripheral face, stacking recesses and a duct having a flow path therein for conveying forming materials (a fiber material and an absorbent polymer) to the stacking recesses. While the rotating drum is rotated about the rotating shaft in the drum circumferential direction, the forming materials conveyed on an air flow generated in the flow path by aspiration from the inside of the rotating drum are stacked on the stacking recesses. The stacked product formed in the stacking recess through such a stacking process is the first layer 6.

The second layer 7 includes a base sheet 71 and an absorbent polymer P placed on one surface (the skin-facing surface) of the base sheet 71 as shown in Fig. 2.

In the present embodiment, an intermediate sheet 8 is placed between the first layer 6 and the second layer 7, and the base sheet 71 included in the second layer 7 is placed to face the non-skin-facing surface side of the intermediate sheet 8. The absorbent polymer P included in the second layer 7 is placed between the two sheets 8 and 71 placed to face together in the thickness direction. The intermediate sheet 8 is a member different from the first layer 6 and the second layer 7.

An absorbent polymer placement layer (the layer between two sheets 8 and 71 in the embodiment) in the second layer 7 typically contains only an absorbent polymer as an absorbent material and contains no absorbent fiber material such as cellulose fibers or, if containing an absorbent fiber material, contains the absorbent fiber material at a smaller content (for example, 10% by mass or less, preferably 3% by mass or less, relative to the total mass of the absorbent polymer placement layer) than the content of the absorbent fiber material in the first layer 6. As the absorbent polymer included in the second layer 7, a similar absorbent polymer to the absorbent polymer included in the first layer 6 may be used.

In order to join two sheets 8 and 71 or to join the sheet 8 or 71 and the absorbent polymer, an adhesive such as a hot melt adhesive may be applied onto the surface (inner surface) that is of at least one of the sheets 8 and 71 and is to face the absorbent polymer placement layer.

As the base sheet 71 included in the second layer 7, a liquid permeable or liquid absorbable sheet may be used. The base sheet 71 is typically a fiber sheet mainly containing fibers or having a fiber content of more than 50% by mass.

Examples of the constituent fibers in the base sheet 71 include natural fibers such as wood pulps including softwood pulp and hardwood pulp and non-wood pulps including cotton pulp and hemp pulp; modified pulps such as cationized pulp and mercerized pulp (i.e., cellulose fibers); and synthetic fibers containing a resin such as polyethylene and polypropylene, and these materials may be used singly or in combination of two or more of them.

Example forms of the base sheet 71 include paper, woven fabric, and nonwoven fabric, and examples of the nonwoven fabric include air-through nonwoven fabric, heat roll nonwoven fabric, spunlace nonwoven fabric, spunbond nonwoven fabric, meltblown nonwoven fabric, and spunbond-meltblown-spunbond (SMS) nonwoven fabric. The base sheet 71 typically has a single-layer structure formed from one of these materials but may have a laminate structure in which two or more materials are laminated and integrated.

The basis weight of the base sheet 71 is not specifically limited but is preferably 5 g/m² or more and more preferably 8 g/m² or more and is preferably 50 g/m² or less and more preferably 30 g/m² or less from the viewpoint of allowing the second layer 7 to certainly achieve intended effects.

As the intermediate sheet 8 separating the first layer 6 and the second layer 7 in the thickness direction, a liquid permeable or liquid absorbable sheet may be used, and the same sheet as the base sheet 71 may be used. The above description for the base sheet 71 is appropriately applied to the intermediate sheet 8.

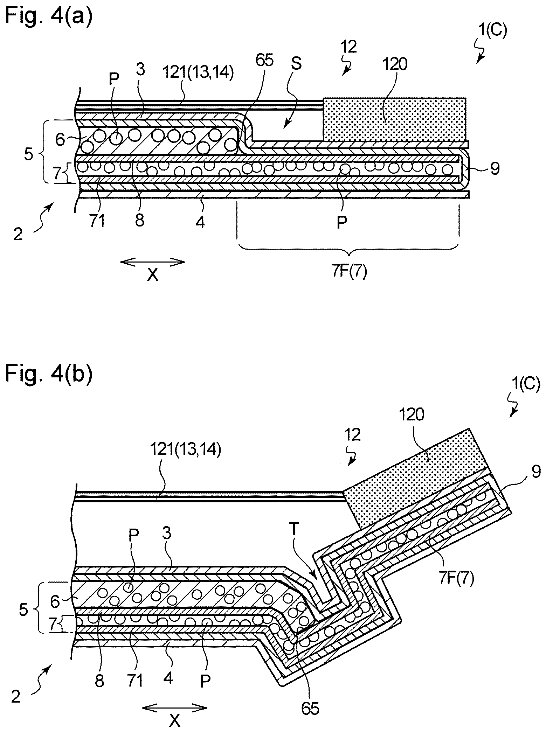

Figs. 4 show the end portion fixing part 120 of the leak-proof cuff 12 in the rear portion C and the vicinity thereof. In the rear portion C of the diaper 1, the end portion fixing part 120 overlaps with the longitudinal extension 7F of the second layer 7 in plan view, and the longitudinal end 65 of the first layer 6 or an imaginary extension line of the longitudinal end 65 is located longitudinally inward from the end portion fixing part 120 and is located a space S apart from the end portion fixing part 120. In the rear portion C of the diaper 1, as shown in Fig. 1, two end portion fixing parts 120 are present, and accordingly two spaces S are present. The diaper 1 has no space S in the front portion A, but in the present invention, spaces S may be present in the front portion A and/or the rear portion C as described later.

The longitudinal extension 7F of the second layer 7 has a smaller bending rigidity than the first layer 6 (the bending rigidity of the longitudinal extension 7F < the bending rigidity of the first layer 6). In other words, the longitudinal extension 7F of the second layer is more flexible and is more easily deformed than the first layer 6.

Typically, each member (the first layer 6, the second layer 7, the intermediate sheet 8, the core-wrap sheet 9) included in the absorbent member 5 has a uniform bending rigidity in the whole member, and the second layer 7 has substantially no difference in bending rigidity between the longitudinal extension 7F and the other portion (the portion overlapping with the first layer 6 in plan view). Accordingly, the second layer 7 is typically considered to have a smaller bending rigidity than the first layer 6. When the first layer 6 partially has different bending rigidities, and a portion in the first layer 6 near the longitudinal extension 7F of the second layer 7 (specifically, for example, a portion located inside in longitudinal direction X from the longitudinal end 65 of the first layer 6 and having a length not more than 25% of the entire length in the longitudinal direction X of the first layer 6) has a larger bending rigidity than the bending rigidity of the longitudinal extension 7F, the magnitude relation "the longitudinal extension 7F of the second layer 7 < the first layer 6" in terms of the bending rigidity is satisfied.

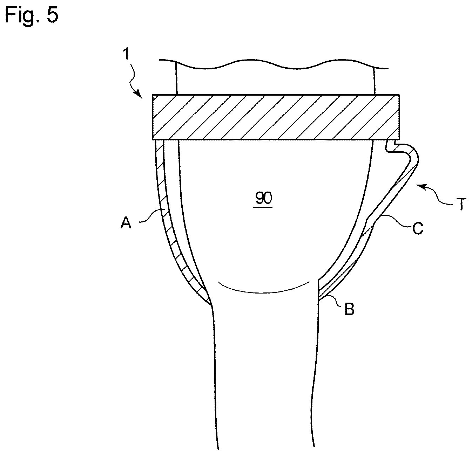

As for the end portion fixing part 120 of the leak-proof cuff 12 in the rear portion C and the vicinity thereof in the diaper 1 having the above structure, when stretched elastic members (for example, the elastic members 14, 15 or 16) in portions in the diaper 1 are released from the diaper 1 (in an unworn state) in a spread out and stretched state shown in Fig. 4(a), specifically, for example, when the diaper 1 is in a worn state or in a natural state, especially the shrinkage force of the stretch portions 121 (leak-proof cuff-forming elastic members 14) in the leak-proof cuffs 12 allows the stretch portions 121 to rise toward the skin of a wearer and to pull the end portion fixing parts 120 in the front portion A and the rear portion C toward the crotch portion B as shown in Fig. 4(b). In such a diaper, in the space S between the end portion fixing part 120 and the adjacent longitudinal end 65 of the first layer 6, the longitudinal extension 7F of the second layer 7 having a smaller bending rigidity than the first layer 6 is present, but no first layer 6 is present. Hence, the two spaces S and S in the rear portion C and a portion between them function as a bending guide portion, and a portion located outside in the longitudinal direction X from the longitudinal end 65 of the first layer 6, including the longitudinal extension 7F of the second layer 7, bends toward the skin-facing surface side of the diaper 1 (the side of the topsheet 3). In the bending portion, a pocket structure T concave on the skin-facing surface side (convex on the non-skin-facing surface side) is formed (see Fig. 5). In the pocket structure T (in the concave on the skin-facing surface side), at least a part of the longitudinal extension 7F of the second layer 7 is located, and the end portion in the longitudinal direction X of the first layer 6 (the longitudinal end 65 and the vicinity thereof) may be further located as shown in Fig. 4(b).

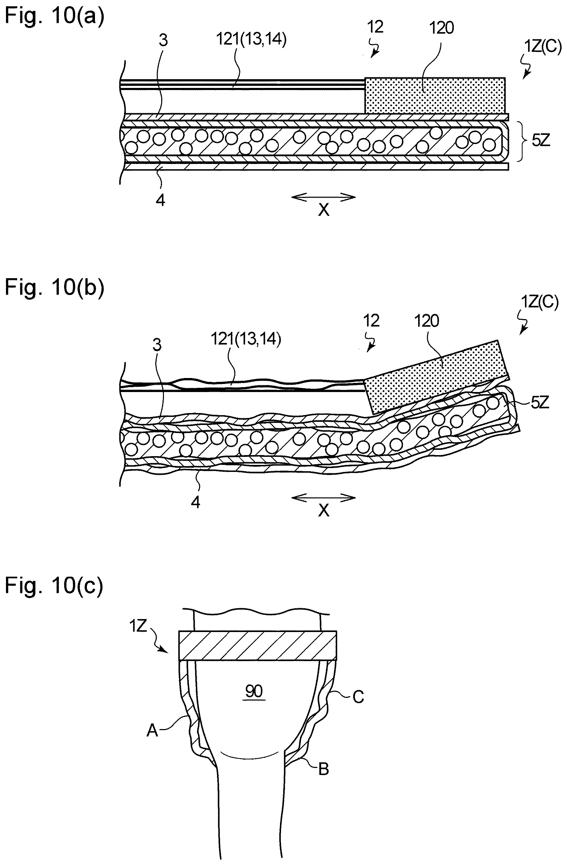

Figs. 10 are views showing a diaper 1Z as an example of a conventional disposable diaper and corresponding to Figs. 4 and Fig. 5. The diaper 1Z has basically the same structure as the diaper 1 except that an absorbent member 5Z is included in place of the absorbent member 5 having a laminate structure. The absorbent member 5Z has a single-layer structure and is a stacked absorbent member or a sheet-type absorbent member. In the diaper 1Z, as shown in Fig. 10(a), the end portion fixing part 120 of the leak-proof cuff 12 is placed on the skin-facing surface of the absorbent member 5Z having a single-layer structure, and the space S provided in the diaper 1, or the space between the end portion fixing part 120 and the longitudinal end of the layer (first layer) at the skin-facing surface side of the absorbent member having a laminate structure is not provided. In the diaper 1Z having such a structure, when the stretch portion 121 (leak-proof cuff-forming elastic members 14) of each leak-proof cuff 12 shrinks at the time of wearing, the shrinkage force is stronger than the rigidity of the absorbent member 5Z, and consequently irregular creases are formed on the whole outer surface in a portion with the absorbent member 5Z in the diaper 1Z as shown in Fig. 10(b) and Fig. 10(c). The creases may cause deteriorations in leak-proof properties, fitting properties, appearance, and other properties. In particular, when a sheet-type absorbent member is used as the absorbent member 5Z, for example, to slim down the absorbent article, the diaper 1Z is weak in firmness and easily twists, and such concerns can be more serious.

In contrast, in the diaper 1 in a worn state, as described above, the space S between the end portion fixing part 120 of the leak-proof cuff 12 and the first layer 6 functions as a bending guide portion, and the diaper 1 bends at the space S. Hence, the shrinkage force of the stretch portion 121 in each leak-proof cuff 12 is unlikely to affect the absorbent member 5, and the disadvantages concerned in the conventional diaper 1Z are unlikely to be caused. In the diaper 1, the pocket structure T formed at the position of the space S by the bending diaper 1 functions as a pocket that temporarily stores a body fluid such as urine, and thus leakage in the longitudinal direction X can be effectively prevented. As described above, in the diaper 1, the leak-proof cuffs 12 can prevent lateral leakage. In addition, the pocket structures T are formed at the positions of the spaces S when the diaper is worn and can prevent leakage in the longitudinal direction X. Hence, the diaper has excellent leakage prevention performance.

In addition, in the diaper 1, the longitudinal extension 7F of the second layer 7 has a higher absorbent polymer density than the first layer 6 (the absorbent polymer density of the longitudinal extension 7F > the absorbent polymer density of the first layer 6). Typically, a member having a higher absorbent polymer density tends to have a lower liquid diffusion speed. Hence, when the magnitude relation "the longitudinal extension 7F of the second layer 7 > the first layer 6" in terms of absorbent polymer density is satisfied, the longitudinal extension 7F has a smaller liquid diffusion speed than the first layer 6, and the absorbent member 5 can achieve more efficient liquid absorption as a whole. Achieving such efficient use of the absorbent member 5 can greatly contribute to an improvement in leakage prevention performance of the diaper 1.

Typically, each of the first layer 6 and the second layer 7 has a uniform absorbent polymer density throughout the layer. Accordingly, the second layer 7 is typically thought to have a higher absorbent polymer density than the first layer 6. The first layer 6 may contain no absorbent polymer. In such a case, the first layer 6 has an absorbent polymer density of 0, but the second layer 7 certainly contains an absorbent polymer, and thus the magnitude relation is satisfied.

In particular, in the present embodiment, as shown in Figs. 4, the intermediate sheet 8 is placed between the first layer 6 and the second layer 7, the intermediate sheet 8 has a longitudinal extension extending longitudinally outward from the first layer 6 in at least one of the front portion A and the rear portion C (in the rear portion C in the embodiment shown in figures), and the longitudinal extension of the intermediate sheet 8 overlaps with the longitudinal extension 7F of the second layer 7. More specifically, in the present embodiment, the intermediate sheet 8 has the same shape and dimensions as the second layer 7 in plan view, overlies the whole region of the skin-facing surface (the face facing the first layer 6) of the second layer 7, and has, in the rear portion C, the longitudinal extension extending longitudinally outward from the longitudinal end 65 (the tangent 65L) of the first layer 6. When the diaper 1 is worn, a part of the longitudinal extension of the intermediate sheet 8 is located in the pocket structure T (in the concave on the skin-facing surface side) together with the longitudinal extension 7F of the second layer 7 (see Fig. 4(b)). The intermediate sheet 8 has liquid permeability or liquid absorbability as described above. Hence, such a structure further improves the liquid diffusibility of the absorbent member 5 to lead to efficient use of the absorbent member 5, enabling further improvement in leakage prevention performance of the diaper 1.

In the present embodiment, the first layer 6 is what is called a stacked absorbent member in which a stack of a fiber material supports an absorbent polymer, whereas the second layer 7 is what is called a sheet-type absorbent member including a base sheet 71 placed to face an intermediate sheet 8 and an absorbent polymer placed between the sheets 8and 71. Hence, the magnitude relation in terms of bending rigidity (the bending rigidity of the longitudinal extension 7F of the second layer 7 < the bending rigidity of the first layer 6) is easily satisfied. Accordingly, the diaper 1 more certainly bends at the spaces S, and the pocket structures T are more certainly formed.

In the present embodiment, as shown in Fig. 1, the longitudinal end 65 of the first layer 6 from which the longitudinal extension 7F of the second layer 7 extends (i.e., the longitudinal end 65 in the rear portion C) is coincident in the longitudinal direction X with the longitudinal end of the stretch portion 121 of the leak-proof cuff 12. This allows the diaper 1 to easily bend at the spaces S, and accordingly the pocket structures T are easily formed. In the description, the "being coincident" means the following condition: in such a diaper 1 in a spread out and stretched state as shown in Fig. 1, the ratio of L1 to L0 is 95 to 105% where L1 is the length in the longitudinal direction X from the article longitudinal end X1 to the longitudinal end of the stretch portion 121 of the leak-proof cuff 12 (i.e., the longitudinal end at the side of the space S), L0 is the length in the longitudinal direction X from the article longitudinal end X1 to the longitudinal end 65, and the article longitudinal end X1 is the end in the longitudinal direction X of the diaper 1 in a portion with the longitudinal end 65 of the first layer 6 (in the rear portion C in the embodiment in Fig. 1).

In the present embodiment, as described above, as shown in Fig. 1 and Fig. 3, the second layer 7 has the longitudinal extension 7F in the rear portion C and has the lateral extensions 7E extending laterally outward from the side edges along the longitudinal direction X of the first layer 6, and the longitudinal extension 7F in the rear portion C and the lateral extensions 7E form the extension having a U shape in plan view. It is preferable that 70% or more, preferably 80% or more, of the area of the stretch portions 121 of the leak-proof cuffs 12 overlap with the extension having a U shape in plan view. Such a structure allows the diaper 1 to more easily bend at the spaces S, and accordingly the pocket structures T are more easily formed. When a wearer wearing the diaper 1 is specifically a baby, the fitting properties to the body of the baby can be further improved in the rear portion C. In the description, "the area of the stretch portion 121" typically means the area of a region between the end portion fixing part 120 in the front portion A and the end portion fixing part 120 in the rear portion C in a leak-proof cuff 12 located at the same side of the longitudinal center line CLx.

From the viewpoint of certainly satisfying the magnitude relation (the longitudinal extension 7F < the first layer 6) in terms of the bending rigidity and of more certainly forming the pocket structures T, the bending rigidity of each layer included in the absorbent member 5 is preferably set at the following values. The bending rigidity of each layer 6 and 7 included in the absorbent member 5 may be adjusted by controlling the material, the density, the thickness, and the like of the layer or by subjecting the layer to secondary processing, and examples of the secondary processing include processing in which a groove (slit) is formed on the layer to help a portion with the groove to bend.

The bending rigidities of the absorbent member 5 and constituent members of the absorbent member 5, such as the first layer 6 and the second layer 7, may be evaluated by bending rigidity values determined by the following method. A sample having a smaller bending rigidity value is considered to have a lower bending rigidity (higher flexibility).

The ratio of the bending rigidity of the longitudinal extension 7F of the second layer 7 to the bending rigidity of the first layer 6, in terms of the former/the latter, is preferably 0.1 or more and more preferably 0.15 or more and is preferably 0.4 or less and more preferably 0.35 or less provided that the former < the latter.

The bending rigidity of the longitudinal extension 7F of the second layer 7 is preferably 5.0 gf or more and more preferably 7.5 gf or more and is preferably 50 gf or less and more preferably 35 gf or less provided that the bending rigidity of the longitudinal extension 7F of the second layer 7 is smaller than the bending rigidity of the first layer 6.

A portion in the second layer 7 other than the longitudinal extension 7F (a portion overlapping with the first layer 6 in plan view) may have substantially the same bending rigidity as that of the longitudinal extension 7F.

The bending rigidity of the first layer 6 is preferably 40 gf or more and more preferably 50 gf or more and is preferably 200 gf or less and more preferably 180 gf or less provided that the bending rigidity of the first layer 6 is larger than the bending rigidity of the longitudinal extension 7F of the second layer 7.

### <Measurement method of bending rigidity>

The measurement method may be performed by using a handle-o-meter. The measurement method with a handle-o-meter is performed in accordance with Japanese Industrial Standards "JIS L-1096 (General Testing Method for Woven fabrics)". A measurement object (a first layer 6, a second layer 7) is cut into a measurement sample having a quadrangular shape in plan view with a dimension of 100 mm in the absorbent article longitudinal direction and a dimension of 50 mm in the absorbent article lateral direction. The measurement sample is placed on a support table having a groove 10 mm in width such that the direction corresponding to the longitudinal direction is orthogonal to the groove extending direction. The center of the measurement sample is pressed with a blade 2 mm in thickness, and the resistance value (g) when the measurement sample is pushed in 8 mm is determined by using a load cell. The average of five points is calculated as the measured value. As the measurement apparatus, for example, a texture meter (handle-o-meter method), type HOM-2, manufactured by DAIEI KAGAKU SEIKI may be used.

From the viewpoint of certainly satisfying the magnitude relation in terms of the bending rigidity (the longitudinal extension 7F < the first layer 6) and of more certainly forming the pocket structures T, the length in the longitudinal direction X of the space S (the distance between the end portion fixing part 120 of the leak-proof cuff 12 and the longitudinal end 65 of the first layer 6) is preferably 1.0 mm or more and more preferably 5.0 mm or more and is preferably 40.0 mm or less and more preferably 30.0 mm or less, in such a diaper 1 in a spread out and stretched state as shown in Fig. 1.

From the viewpoint of certainly satisfying the magnitude relation in terms of the absorbent polymer density (the longitudinal extension 7F > the first layer 6) and of more certainly allowing the absorbent member 5 to achieve efficient liquid absorption, the absorbent polymer density of each layer included in the absorbent member 5 is preferably set at the following values.

The ratio of the absorbent polymer density of the longitudinal extension 7F of the second layer 7 and the absorbent polymer density of the first layer 6, in terms of the latter/the former, is preferably 0.05 or more and more preferably 0.07 or more and is preferably 0.5 or less and more preferably 0.4 or less.

The absorbent polymer density of the longitudinal extension 7F of the second layer 7 is preferably 1.0 g/cm³ or more and more preferably 1.2 g/cm³ or more and is preferably 2.0 g/cm³ or less and more preferably 1.8 g/cm³ or less provided that the absorbent polymer density of the longitudinal extension 7F of the second layer 7 is larger than the absorbent polymer density of the first layer 6.

A portion in the second layer 7 other than the longitudinal extension 7F (a portion overlapping with the first layer 6 in plan view) may have substantially the same absorbent polymer density as that of the longitudinal extension 7F.

The absorbent polymer density of the first layer 6 is preferably 0.05 g/cm³ or more and more preferably 0.07 g/cm³ or more and is preferably 1.0 g/cm³ or less and more preferably 0.8 g/cm³ or less provided that the absorbent polymer density of the first layer 6 is smaller than the absorbent polymer density of the longitudinal extension 7F of the second layer 7.

The absorbent polymer density of each layer (the first layer 6 and the second layer 7) included in the absorbent member 5 may be determined by the following method.

### <Measurement method of absorbent polymer density>

For the above diaper 1 as an example, first, five absorbent assemblies 2 as the measurement object are prepared. From each absorbent assembly 2, members other than the absorbent member 5, such as the topsheet 3, are removed, and the prepared absorbent member 5 is placed on a horizontal place without forming any creases or folding. From each layer (the first layer 6 and the second layer 7) to be measured in the absorbent member 5, a regular square region in plan view having a dimension of 70 mm in the longitudinal direction X and a dimension of 70 mm in the lateral direction Y is cut out as a measurement sample. The thickness of the measurement sample is determined under a load of 0.6 g/cm². The thickness of the measurement sample may be determined, for example, by using a laser displacement meter (LK-080) manufactured by Keyence Corporation. To determine the thickness of a measurement sample, a regular square plate adjusted to give a load of 0.6 g/cm² (a 50 × 50 mm acrylic plate having a thickness of about 5 mm) is placed on the measurement sample, and the thickness of the measurement sample is determined by using the above laser displacement meter. The thickness of the measurement sample is determined at any five points in the surface direction by the above method, and the average is calculated as the thickness of the measurement sample (the first layer 6 and the second layer 7). The absorbent polymer basis weight of each of the layers 6 and 7 is divided by the thickness of the corresponding layer 6 and 7 to give an intended absorbent polymer density of the first layer 6 or the second layer 7.

The absorbent polymer basis weight of the longitudinal extension 7F of the second layer 7 is preferably 55 g/m² or more and more preferably 70 g/m² or more and is preferably 450 g/m² or less and more preferably 300 g/m² or less. A portion in the second layer 7 other than the longitudinal extension 7F (a portion overlapping with the first layer 6 in plan view) may have substantially the same absorbent polymer basis weight as that of the longitudinal extension 7F.

The absorbent polymer basis weight of the first layer 6 is preferably 50 g/m² or more and more preferably 60 g/m² or more and is preferably 150 g/m² or less and more preferably 120 g/m² or less.

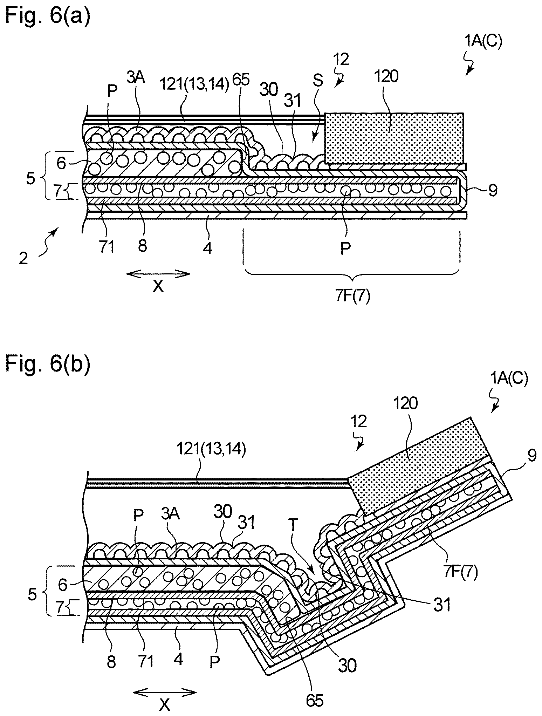

Figs. 6 to Fig. 9 show other embodiments of the absorbent article of the present invention and principal parts thereof. In the other embodiments described below, components different from those in the diaper 1 described above will be mainly described, and a similar component is indicated by an identical sign and is not described. To components not specifically described in the other embodiments described below, the description for the above diaper 1 is appropriately applied.

In a diaper 1A shown in Figs. 6, on the skin-facing surface of a topsheet 3A, a proj ecting-and-depressed region containing a plurality of projections 30 projecting toward the skin of a wearer and depressions 31 located around the projections 30 is formed, and the projecting-and-depressed region is present in the space S between the end portion fixing part 120 of the leak-proof cuff 12 and the longitudinal end 65 of the first layer 6. More specifically, the topsheet 3A has a longitudinal extension longitudinally extending outward from the longitudinal end 65 of the first layer 6 in the rear portion C, and the longitudinal extension of the topsheet 3A together with the longitudinal extension 7F of the second layer 7 and the longitudinal extension of the intermediate sheet 8 reaches the end in the longitudinal direction X of the diaper 1 through the space S. At least on the skin-facing surface of the longitudinal extension of the topsheet 3A and a portion of the topsheet 3A near the longitudinal extension, a projecting-and-depressed region containing a plurality of projections 30 and depressions 31 is formed. The projecting-and-depressed region may be formed on the whole region of the skin-facing surface of the topsheet 3A.

In the end portion fixing part 120 of the leak-proof cuff 12 in the rear portion C and the vicinity thereof in the diaper 1A, when stretched elastic members in portions in the diaper 1A are released from the diaper 1A in a spread out and stretched state (in an unworn state) as shown in Fig. 6(a), specifically, for example, when the diaper 1A is in a worn state or in a natural state, the space S functions as a bending guide portion as with the diaper 1, and a portion in the diaper 1A located outside in the longitudinal direction X from the longitudinal end 65 of the first layer 6 bends toward the skin-facing surface side. Accordingly, a pocket structure T is formed in the bending portion (see Fig. 6(b)). A difference from the diaper 1 is that projections 30 of the topsheet 3A are located in the pocket structure T (in the concave on the skin-facing surface side). The projections 30 have liquid absorbability, and thus such a structure can further improve leakage prevention performance in the longitudinal direction X.

The projecting-and-depressed pattern (the shape and the arrangement of projections) of the projecting-and-depressed region on the skin-facing surface of the topsheet 3A is not specifically limited, and any pattern may be appropriately selected without departing from the scope of the present invention.

Examples of the planar shape of the projection include a circular shape, an elliptical shape, a triangular shape, and a polygonal shape including a quadrangular shape. The projection may have a solid structure filled with constituent fibers of the topsheet therein or may have a hollow structure not filled with the constituent fibers.

An example of the projecting-and-depressed pattern of the projecting-and-depressed region is a pattern in which a plurality of projections are arranged in a scattered manner (for example, in a staggered manner), and depressions are arranged around the projections.

Another example of the projecting-and-depressed pattern of the projecting-and-depressed region is a pattern in which first linear depressions in plan view extending in a first direction intersecting both the longitudinal direction and the lateral direction and second linear depressions in plan view extending in a second direction intersecting the first direction are arranged in a grid pattern, and projections are present in a plurality of sections surrounded by the depressions.

Still another example of the projecting-and-depressed pattern of the projecting-and-depressed region is a pattern in which projections as ridges extending in the longitudinal direction or the lateral direction and depressions as grooves extending in the same direction are alternately arranged in the direction orthogonal to the extending direction.

The non-skin-facing surface of the topsheet 3A is typically a flat face substantially having neither projections nor depressions as shown in Figs. 6.

The method of forming the projecting-and-depressed region of the topsheet 3A is not specifically limited. The projecting-and-depressed region is typically formed by subjecting an original fabric sheet as the material of the topsheet to partial compressing. In such a case, in compressed portions, a forming material of the original fabric sheet is compacted into depressions, whereas uncompressed portions project to one side in the thickness direction, specifically to the skin-facing surface side, into projections. In this case, the projections without compressing are low-density portions having a relatively low density, whereas the depressions with compressing are high-density portions having a relatively high density. For the compressing, any known method may be used, and examples include embossing with or without heat and ultrasonic embossing.

The topsheet 3A may have a single-layer structure or a laminate structure in which a plurality of layers are laminated in the thickness direction. An example of the topsheet having the laminate structure is a topsheet having a two-layer structure in which two sheets in a lamination state are partially joined, and one of the two sheets (skin-facing sheet) to be closer to the skin of a wearer projects in a direction away from the other sheet (non-skin-facing sheet) in portions other than the joined portions to form projections. As the sheet included in the topsheet, a nonwoven fabric is preferably used. Examples of the nonwoven fabric include air-through nonwoven fabric, heat roll nonwoven fabric, spunlace nonwoven fabric, spunbond nonwoven fabric, meltblown nonwoven fabric, and spunbond-meltblown-spunbond (SMS) nonwoven fabric.

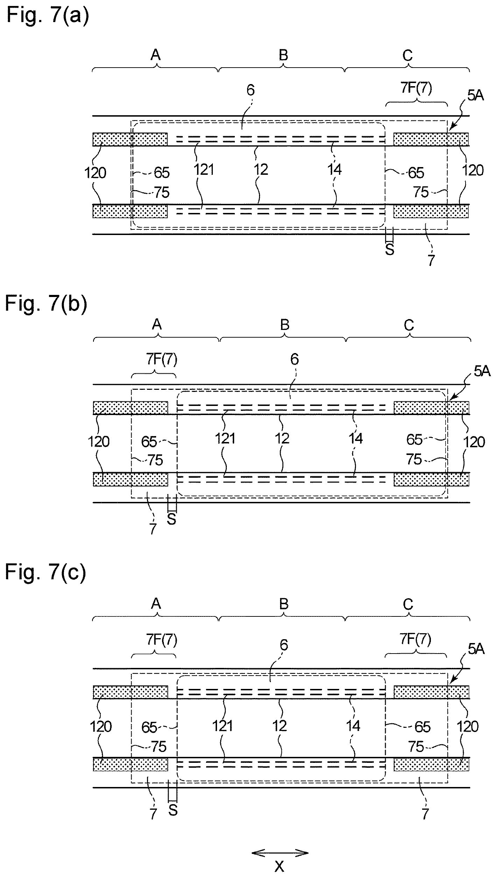

Figs. 7 show arrangements of spaces S applicable in the present invention. In an absorbent member 5A shown in Figs. 7, the first layer 6 has a rectangular shape in plan view; the dimension (width) in the lateral direction Y of the first layer 6 is the same as the second layer 7; and the side edges along the longitudinal direction X of the layer 6 are coincident with those of the layer 7. Accordingly, the second layer 7 of the absorbent member 5Ahas no lateral extension 7E (see Fig. 3).

In an embodiment in Fig. 7(a), the space S is present only in the rear portion C as with the above diaper 1 and is not present in the front portion A. In the front portion A, the longitudinal end 65 of the first layer 6 is coincident with the longitudinal end 75 of the second layer 7 in a portion overlapping with the end portion fixing parts 120 of the leak-proof cuffs 12 in plan view.

In an embodiment in Fig. 7(b), the space S is present only in the front portion A and is not present in the rear portion C. In the rear portion C, the longitudinal end 65 of the first layer 6 is coincident with the longitudinal end 75 of the second layer 7 in a portion overlapping with the end portion fixing parts 120 of the leak-proof cuffs 12 in plan view.

In an embodiment in Fig. 7(c), the spaces S are present in both the front portion A and the rear portion C.

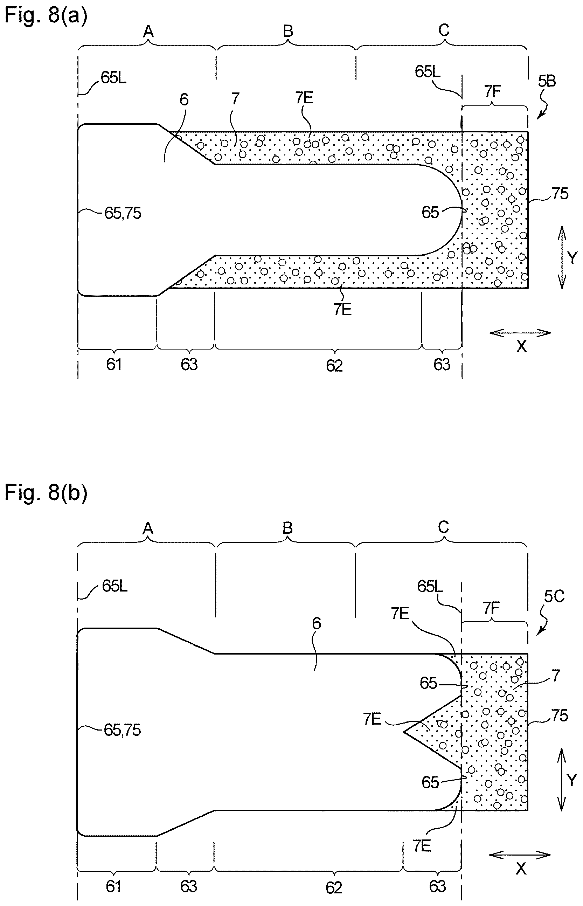

In an absorbent member 5B shown in Fig. 8(a), the longitudinal end 65 of the first layer 6 in the rear portion C is a curved line, more specifically, is a curved line convex longitudinally outward, which differs from the absorbent member 5 (see Fig. 3) in which the longitudinal end 65 is a straight line extending in the lateral direction Y. In the absorbent member 5B, a portion of the first layer 6 with the curved longitudinal end 65 is a variable width portion 63 having widths that gradually decrease from the inside to the outside in the longitudinal direction X (from the side of the crotch portion B to the side of the rear portion C).

An absorbent member 5C shown in Fig. 8(b) has a plurality of (specifically two) longitudinal ends 65 in the rear portion C of the first layer 6. In the absorbent member 5C, the plurality of longitudinal ends 65 are located at the same position in the longitudinal direction X, and the plurality of longitudinal ends 65 have a common tangent 65L. In the first layer 6 of the absorbent member 5C in plan view as shown in Fig. 8(b), portions corresponding to the plurality of (two) longitudinal ends 65 in the rear portion C are convex portions, whereas a portion between the plurality of longitudinal ends 65 is a concave portion, and a lateral extension 7E of the second layer 7 is located in the concave portion.

In an absorbent member 5D shown in Fig. 9, the first tangent 65L is coincident with a second tangent (not shown) extending in the lateral direction Y, to the longitudinal end 75 of the second layer 7 in each of the front portion A and the rear portion C, and the longitudinal extension 7F of the second layer 7 includes portions located at nook portions 50 of the absorbent member 5D defined by the coincident tangents (the first tangents 65L and the second tangents).

More specifically, the absorbent member 5D has a rectangular shape in plan view and has two nook portions 50 in each of the front portion A and the rear portion C (four in total), and a longitudinal extension 7F is present in each nook portion 50. In each longitudinal extension 7F in the four nook portions 50, the above space S is provided. Hence, in an absorbent article (disposable diaper) including the absorbent member 5D, the spaces S may be present in each of the front portion A and the rear portion C.

In the absorbent member 5D, the second layer 7 has a rectangular shape in plan view and has a constant dimension (width) in the lateral direction Y over the entire length in the longitudinal direction X of the second layer 7, whereas the first layer 6 has varying widths and includes maximum width portions 61 having the maximum width and a minimum width portion 62 having the minimum width in the first layer 6, as shown in Fig. 9. More specifically, in the absorbent member 5D, the first layer 6 includes, in each of the front portion A and the rear portion C, a single maximum width portion 61 that is located a certain distance apart from the end in the longitudinal direction X of the first layer 6 inward in the longitudinal direction X, includes a single minimum width portion 62 between the maximum width portion 61 in the front portion A and the maximum width portion 61 in the rear portion C, and further includes, between one maximum width portion 61 and the minimum width portion 62 and between the other maximum width portion 61 and the minimum width portion 62, variable width portions 63 having widths that gradually increase from the inside to the outside in the longitudinal direction X (from the side of the crotch portion B to the side of the front portion A or to the side of the rear portion C). Each of the pair of lateral extensions 7E and 7E of the second layer 7 extends laterally outward from the minimum width portion 62 of the first layer 6 and from two variable width portions 63 and 63 continuing to the respective ends in the longitudinal direction X of the minimum width portion 62 and has a substantially trapezoidal shape in plan view. In the absorbent member 5D, the lateral extensions 7E are located at least in the crotch portion B and may further extend to the front portion A and/or the rear portion C.

The present invention has been described on the basis of the preferred embodiments. A portion only in one of the embodiments can be appropriately used exchangeably for another portion.

The absorbent article of the present invention is not limited to such an open type disposable diaper as in the above embodiments and widely encompasses articles used for absorption of body fluids excreted from a human body (such as urine, menstrual blood, loose feces, and sweat), and pull-on disposable diapers, sanitary napkins, sanitary shorts, not having an attachment structure such as an attachment member 18 and an attachment region 19 are also encompassed.

### Industrial Applicability

According to the present invention, an absorbent article having excellent liquid absorbability in the longitudinal direction, unlikely to cause excrement leakage from the longitudinal end portion, and having excellent wearing comfort is provided.

## Claims

1. An absorbent article (1) that has a longitudinal direction corresponding to a front-rear direction of a wearer and a lateral direction orthogonal to the longitudinal direction and is sectioned into a crotch portion (B) to be placed on a crotch section of a wearer, a front portion (A) to be placed closer to a front side of the wearer than the crotch portion (B), and a rear portion (C) to be placed closer to a rear side of the wearer than the crotch portion (B),
the absorbent article (1)comprising:
an absorbent assembly (2); and
leak-proof cuffs (12) placed at respective sides along the longitudinal direction of the absorbent assembly (2), wherein
the absorbent assembly (2) includes:
an absorbent member (5) to absorb and retain a body fluid; and
a topsheet (3) placed on a skin-facing surface side of the absorbent member (5),
each leak-proof cuff (12) has, in the front portion and the rear portion, end portion fixing parts (120) in which longitudinal end portions of the leak-proof cuff (12) are fixed to the absorbent assembly (2), a portion of the leak-proof cuff (12) between the end portion fixing part (120) in the front portion and the end portion fixing part (120) in the rear portion is a stretch portion (121) that is not fixed to another member and has elasticity in the longitudinal direction,
the absorbent member (5) includes:
a first layer (6); and
a second layer (7) placed on a non-skin-facing surface side of the first layer (6) and containing an absorbent polymer (P), the second layer (7) having, in at least one of the front portion and the rear portion, a longitudinal extension (7F) extending longitudinally outward from the first layer (6),
wherein a first tangent (65L) extends in the lateral direction (Y) to a longitudinal end (65) of the first layer (6); and
the longitudinal extension (7F) of the second layer (7)
includes 1) a portion extending longitudinally outward from the first tangent (65L) or
includes 2), when the first tangent (65L) is coincident with a second tangent that extends in the lateral direction (Y), to a longitudinal end (75) of the second layer (7), a portion defined by the coincident first and second tangent and located at a nook portion (50) of the absorbent member (5),
in at least one of the front portion (A) and the rear portion (C), the end portion fixing part (120) overlaps with the longitudinal extension (7F) of the second layer (7) in plan view, the longitudinal end (65) of the first layer (6) is located longitudinally inward from the end portion fixing part (120) and is located a space apart from the end portion fixing part (120), and
the second layer (7) has the longitudinal extension (7F) in the rear portion (C) and has lateral extensions (7E) extending laterally outward from side edges along the longitudinal direction of the first layer (6), the longitudinal extension (7F) in the rear portion (C) and the lateral extensions (7E) form an extension having a U shape in plan view,
70% or more of an area of the stretch portions (121) of the leak-proof cuffs (12) overlaps with the extension having a U shape in plan view, and
the longitudinal extension (7F) of the second layer (7) has a smaller bending rigidity than the first layer (6) and has a larger density of the absorbent polymer (P) than the first layer (6).

2. The absorbent article (1) according to claim 1, wherein an intermediate sheet (8) is placed between the first layer (6) and the second layer (7), the intermediate sheet (8) has, in at least one of the front portion (A) and the rear portion (C), a longitudinal extension extending longitudinally outward from the first layer (6), and the longitudinal extension of the intermediate sheet (8) overlaps with the longitudinal extension (7F) of the second layer (7).

3. The absorbent article (1) according to claim 2, wherein the first layer (6) has a structure in which a stack of a fiber material supports an absorbent polymer (P), and
the second layer (7) includes a base sheet (71) placed to face the intermediate sheet (8) and the absorbent polymer (P) placed between the sheets.

4. The absorbent article according to any one of claims 1 to 3, wherein on a skin-facing surface of the topsheet (3), a projecting-and-depressed region containing a plurality of projections projecting toward a skin of a wearer is formed, and the projecting-and-depressed region is present in the space (S) between the end portion fixing part and the longitudinal end (65) of the first layer (6).

5. The absorbent article (1) according to any one of claims 1 to 4, wherein the longitudinal end (65) of the first layer (6) from which the longitudinal extension (7F) of the second layer (7) extends is coincident in the longitudinal direction with a longitudinal end of the stretch portion (121) of the leak-proof cuff (12).

6. The absorbent article (1) according to any one of claims 1 to 5, wherein the longitudinal extension (7F) of the second layer (7) is present in the front portion (A).

7. The absorbent article (1) according to any one of claims 1 to 6, wherein the longitudinal extension (7F) of the second layer (7) is present in the rear portion (C).

8. The absorbent article (1) according to any one of claims 1 to 7, wherein in the first layer (6), the absorbent polymer (P) is uniformly distributed throughout the first layer (6).

9. The absorbent article (1) according to any one of claims 1 to 7, wherein in the first layer (6), the absorbent polymer (P) is contained at a larger content in one of the skin-facing surface side and the non-skin-facing surface side than in the other.

10. The absorbent article (1) according to any one of claims 1 to 9, wherein a ratio of the bending rigidity of the longitudinal extension (7F) of the second layer (7) to the bending rigidity of the first layer (6), in terms of the former/the latter, is 0.1 or more and 0.4 or less, and
the bending rigidity of the longitudinal extension (7F) of the second layer (7) is 5.0 gf or more and 50 gf or less.

11. The absorbent article (1) according to any one of claims 1 to 10, wherein a ratio of the absorbent polymer (P) density of the longitudinal extension (7F) of the second layer (7) and the absorbent polymer (P) density of the first layer (6), in terms of the latter/the former, is 0.05 or more and 0.5 or less.

12. The absorbent article (1) according to any one of claims 1 to 11, wherein the absorbent article (1) is a disposable diaper.

## Patentansprüche

1. Saugfähiger Artikel (1), der eine einer Vorne-Hinten-Richtung eines Trägers entsprechende Längsrichtung und eine zu der Längsrichtung orthogonale Querrichtung aufweist und in einen auf einen Schrittbereich eines Trägers zu platzierenden Schrittabschnitt (B), einen näher an einer Vorderseite des Trägers als der Schrittabschnitt (B) zu platzierenden vorderen Abschnitt (A) und einen näher an einer Hinterseite des Trägers als der Schrittabschnitt (B) zu platzierenden hinteren Abschnitt (C) unterteilt ist,
wobei der saugfähige Artikel (1) Folgendes umfasst:
eine saugfähige Anordnung (2); und
auslaufsichere Bündchen (12), die an jeweiligen Seiten entlang der Längsrichtung der saugfähigen Anordnung (2) platziert sind, wobei
die saugfähige Anordnung (2) Folgendes beinhaltet:
ein saugfähiges Element (5) zum Aufsaugen und Einbehalten einer Körperflüssigkeit; und
eine Decklage (3), die auf einer der Haut zugewandten Oberfläche des saugfähigen Elements (5) platziert ist,
wobei die auslaufsicheren Bündchen (12) in dem vorderen Abschnitt und dem hinteren Abschnitt jeweils Endabschnittsfixierungsteile (120) aufweisen, in denen Längsendeabschnitte des auslaufsicheren Bündchens (12) an der saugfähigen Anordnung (2) fixiert sind, ein Abschnitt des auslaufsicheren Bündchens (12) zwischen dem Endabschnittsfixierungsteil (120) in dem vorderen Abschnitt und dem Endabschnittsfixierungsteil (120) in dem hinteren Abschnitt ein Dehnungsabschnitt (121) ist, der nicht an einem anderen Element fixiert ist und Elastizität in der Längsrichtung aufweist,
wobei das saugfähige Element (5) Folgendes beinhaltet:
eine erste Schicht (6); und
eine zweite Schicht (7), die auf einer nicht der Haut zugewandten Oberfläche der ersten Schicht (6) platziert ist und ein saugfähiges Polymer (P) enthält, wobei die zweite Schicht (7) in mindestens einem von dem vorderen Abschnitt und dem hinteren Abschnitt einen Längsfortsatz (7F) aufweist, der sich in Längsrichtung von der ersten Schicht (6) nach außen erstreckt,
wobei sich eine erste Tangente (65L) in der Querrichtung (Y) zu einem Längsende (65) der ersten Schicht (6) erstreckt; und
der Längsfortsatz (7F) der zweiten Schicht (7) Folgendes beinhaltet:
1) einen sich in Längsrichtung von der ersten Tangente (65L) nach außen erstreckenden Abschnitt
oder
2) wenn sich die erste Tangente (65L) mit einer zweiten Tangente deckt, die sich in der Querrichtung (Y) zu einem Längsende (75) der zweiten Schicht (7) erstreckt, einen Abschnitt beinhaltet, der von der ersten und der zweiten Tangente, die sich decken, definiert ist und sich an einem Eckabschnitt (50) des saugfähigen Elements (5) befindet,
wobei in mindestens einem von dem vorderen Abschnitt (A) und dem hinteren Abschnitt (C) der Endabschnittsfixierungsteil (120) und der Längsfortsatz (7F) der zweiten Schicht (7) einander in der Draufsicht überlappen, sich das Längsende (65) der ersten Schicht (6) in Längsrichtung weiter innen befindet als der Endabschnittsfixierungsteil (120) und sich um einen Abstand von dem Endabschnittsfixierungsteil (120) entfernt befindet und
wobei die zweite Schicht (7) den Längsfortsatz (7F) in dem hinteren Abschnitt (C) aufweist und sich entlang der Längsrichtung der ersten Schicht (6) von Seitenkanten quer nach außen erstreckende Querfortsätze (7E) aufweist, wobei der Längsfortsatz (7F) in dem hinteren Abschnitt (C) und die Querfortsätze (7E) einen in der Draufsicht eine U-Form aufweisenden Fortsatz bilden,
wobei 70 % oder mehr einer Fläche der Dehnungsabschnitte (121) der auslaufsicheren Bündchen (12) und der in der Draufsicht eine U-Form aufweisende Fortsatz einander überlappen und
wobei der Längsfortsatz (7F) der zweiten Schicht (7) eine geringere Biegesteifigkeit aufweist als die erste Schicht (6) und eine höhere Dichte des saugfähigen Polymers (P) aufweist als die erste Schicht (6).

2. Saugfähiger Artikel (1) nach Anspruch 1, wobei eine Zwischenlage (8) zwischen die erste Schicht (6) und die zweite Schicht (7) platziert ist, die Zwischenlage (8) in mindestens einem von dem vorderen Abschnitt (A) und dem hinteren Abschnitt (C) einen Längsfortsatz aufweist, der sich in Längsrichtung von der ersten Schicht (6) nach außen erstreckt, und der Längsfortsatz der Zwischenlage (8) und der Längsfortsatz (7F) der zweiten Schicht (7) einander überlappen.

3. Saugfähiger Artikel (1) nach Anspruch 2, wobei die erste Schicht (6) einen Aufbau aufweist, in dem ein Stapel eines Fasermaterials ein saugfähiges Polymer (P) trägt, und
die zweite Schicht (7) eine Basislage (71) beinhaltetet, die platziert ist, um der Zwischenlage (8) und dem zwischen den Lagen platzierten saugfähigen Polymer (P) zugewandt zu sein.

4. Saugfähiger Artikel nach einem der Ansprüche 1 bis 3, wobei auf einer der Haut zugewandten Oberfläche der Decklage (3) ein vorstehender und eingedrückter Bereich gebildet ist, der eine Vielzahl von in Richtung einer Haut eines Trägers vorstehenden Vorsprüngen enthält, und der vorstehende und eingedrückte Bereich in dem Zwischenraum (S) zwischen dem Endabschnittsfixierungsteil und dem Längsende (65) der ersten Schicht (6) vorhanden ist.

5. Saugfähiger Artikel (1) nach einem der Ansprüche 1 bis 4, wobei sich das Längsende (65) der ersten Schicht (6), von dem sich der Längsfortsatz (7F) der zweiten Schicht (7) erstreckt, in der Längsrichtung mit einem Längsende des Dehnungsabschnitts (121) des auslaufsicheren Bündchens (12) deckt.

6. Saugfähiger Artikel (1) nach einem der Ansprüche 1 bis 5, wobei der Längsfortsatz (7F) der zweiten Schicht (7) in dem vorderen Abschnitt (A) vorhanden ist.

7. Saugfähiger Artikel (1) nach einem der Ansprüche 1 bis 6, wobei der Längsfortsatz (7F) der zweiten Schicht (7) in dem hinteren Abschnitt (C) vorhanden ist.

8. Saugfähiger Artikel (1) nach einem der Ansprüche 1 bis 7, wobei in der ersten Schicht (6) das saugfähige Polymer (P) in der ganzen ersten Schicht (6) gleichmäßig verteilt ist.

9. Saugfähiger Artikel (1) nach einem der Ansprüche 1 bis 7, wobei in der ersten Schicht (6) das saugfähige Polymer (P) in einer von der der Haut zugewandten Seite und der nicht der Haut zugewandten Seite in einem höheren Gehalt enthalten ist als in der anderen.

10. Saugfähiger Artikel (1) nach einem der Ansprüche 1 bis 9, wobei ein Verhältnis der Biegesteifigkeit des Längsfortsatzes (7F) der zweiten Schicht (7) zu der Biegesteifigkeit der ersten Schicht (6) bezogen auf ersteres/letzteres 0,1 oder mehr und 0,4 oder weniger beträgt und
die Biegesteifigkeit des Längsfortsatzes (7F) der zweiten Schicht (7) 5,0 gf oder mehr und 50 gf oder weniger beträgt.

11. Saugfähiger Artikel (1) nach einem der Ansprüche 1 bis 10, wobei ein Verhältnis der Dichte des saugfähigen Polymers (P) des Längsfortsatzes (7F) der zweiten Schicht (7) zu der Dichte des saugfähigen Polymers (P) der ersten Schicht (6) bezogen auf ersteres/letzteres 0.05 oder mehr und 0.5 oder weniger beträgt

12. Saugfähiger Artikel (1) nach einem der Ansprüche 1 bis 11, wobei der saugfähige Artikel (1) eine Einwegwindel ist.

## Revendications

1. Article absorbant (1) qui présente une direction longitudinale correspondant à une direction avant-arrière d'un porteur et une direction latérale orthogonale à la direction longitudinale et est sectionné en une partie d'entrejambe (B) devant être placée sur une section d'entrejambe d'un porteur, une partie avant (A) devant être placée plus près d'un côté avant du porteur que la partie d'entrejambe (B), et une partie arrière (C) devant être placée plus près d'un côté arrière du porteur que la partie d'entrejambe (B),
l'article absorbant (1) comprenant :
un ensemble absorbant (2) ; et
des revers antifuite (12) placés au niveau de côtés respectifs le long de la direction longitudinale de l'ensemble absorbant (2), dans lequel
l'ensemble absorbant (2) inclut :
un élément absorbant (5) pour absorber et retenir un fluide corporel ; et
une feuille supérieure (3) placée sur un côté de surface faisant face à la peau de l'élément absorbant (5),
chaque revers antifuite (12) présente, dans la partie avant et la partie arrière, des parties de fixation de partie d'extrémité (120) dans lesquelles des parties d'extrémité longitudinale du revers antifuite (12) sont fixées à l'ensemble absorbant (2), une partie du revers antifuite (12) entre la partie de fixation de partie d'extrémité (120) dans la partie avant et la partie de fixation de partie d'extrémité (120) dans la partie arrière est une partie extensible (121) qui n'est pas fixée à un autre élément et présente une élasticité dans la direction longitudinale,
l'élément absorbant (5) inclut :
une première couche (6) ; et
une seconde couche (7) placée sur un côté de surface ne faisant pas face à la peau de la première couche (6) et contenant un polymère absorbant (P), la seconde couche (7) présentant, dans au moins l'une de la partie avant et de la partie arrière, une extension longitudinale (7F) s'étendant longitudinalement vers l'extérieur depuis la première couche (6),
dans lequel une première tangente (65L) s'étend dans la direction latérale (Y) vers une extrémité longitudinale (65) de la première couche (6) ; et
l'extension longitudinale (7F) de la seconde couche (7) inclut :
1) une partie s'étendant longitudinalement vers l'extérieur à partir de la première tangente (65L)
2), lorsque la première tangente (65L) est coïncidente avec une seconde tangente qui s'étend dans la direction latérale (Y), vers une extrémité longitudinale (75) de la seconde couche (7), une partie définie par les première et seconde tangentes coïncidentes et située au niveau d'une partie de recoin (50) de l'élément absorbant (5), dans au moins l'une de la partie avant (A) et de la partie arrière (C), la partie de fixation de partie d'extrémité (120) chevauche l'extension longitudinale (7F) de la seconde couche (7) dans une vue en plan, l'extrémité longitudinale (65) de la première couche (6) est située longitudinalement vers l'intérieur à partir de la partie de fixation de partie d'extrémité (120) et est située espacée de la partie de fixation de partie d'extrémité (120), et
la seconde couche (7) présente l'extension longitudinale (7F) dans la partie arrière (C) et présente des extensions latérales (7E) s'étendant latéralement vers l'extérieur depuis des bords latéraux le long de la direction longitudinale de la première couche (6), l'extension longitudinale (7F) dans la partie arrière (C) et les extensions latérales (7E) forment une extension présentant une forme de U dans une vue en plan,
70 % ou plus d'une zone des parties extensibles (121) des revers antifuite (12) chevauchent l'extension présentant une forme de U dans une vue en plan, et
l'extension longitudinale (7F) de la seconde couche (7) présente une rigidité à la flexion inférieure à celle de la première couche (6) et présente une densité du polymère absorbant (P) supérieure à celle de la première couche (6).

2. Article absorbant (1) selon la revendication 1, dans lequel une feuille intermédiaire (8) est placée entre la première couche (6) et la seconde couche (7), la feuille intermédiaire (8) présente, dans au moins l'une de la partie avant (A) et de la partie arrière (C), une extension longitudinale s'étendant longitudinalement vers l'extérieur depuis la première couche (6), et l'extension longitudinale de la feuille intermédiaire (8) chevauche l'extension longitudinale (7F) de la seconde couche (7).

3. Article absorbant (1) selon la revendication 2, dans lequel la première couche (6) présente une structure dans laquelle une pile d'un matériau fibreux supporte un polymère absorbant (P), et
la seconde couche (7) inclut une feuille de base (71) placée pour faire face à la feuille intermédiaire (8) et au polymère absorbant (P) placé entre les feuilles.

4. Article absorbant selon l'une quelconque des revendications 1 à 3, dans lequel sur une surface faisant face à la peau de la feuille supérieure (3), une région faisant saillie et évidée contenant une pluralité de saillies faisant saillie en direction de la peau d'un utilisateur est formée, et la région faisant saillie et évidée est présente dans l'espace (S) entre la partie de fixation de partie d'extrémité et l'extrémité longitudinale (65) de la première couche (6).

5. Article absorbant (1) selon l'une quelconque des revendications 1 à 4, dans lequel l'extrémité longitudinale (65) de la première couche (6) depuis laquelle l'extension longitudinale (7F) de la seconde couche (7) s'étend est coïncidente dans la direction longitudinale avec une extrémité longitudinale de la partie extensible (121) du revers antifuite (12).

6. Article absorbant (1) selon l'une quelconque des revendications 1 à 5, dans lequel l'extension longitudinale (7F) de la seconde couche (7) est présente dans la partie avant (A).

7. Article absorbant (1) selon l'une quelconque des revendications 1 à 6, dans lequel l'extension longitudinale (7F) de la seconde couche (7) est présente dans la partie arrière (C).

8. Article absorbant (1) selon l'une quelconque des revendications 1 à 7, dans lequel dans la première couche (6), le polymère absorbant (P) est uniformément réparti partout dans la première couche (6).

9. Article absorbant (1) selon l'une quelconque des revendications 1 à 7, dans lequel dans la première couche (6), le polymère absorbant (P) est contenu au niveau d'un contenu plus grand dans l'un du côté de surface faisant face à la peau et du côté de surface ne faisant pas face à la peau que dans l'autre.

10. Article absorbant (1) selon l'une quelconque des revendications 1 à 9, dans lequel un rapport entre la rigidité à la flexion de l'extension longitudinale (7F) de la seconde couche (7) et la rigidité à la flexion de la première couche (6), en termes de premier/dernier, est de 0,1 ou plus et de 0,4 ou moins, et
la rigidité à la flexion de l'extension longitudinale (7F) de la seconde couche (7) est de 5,0 gf ou plus et de 50 gf ou moins.

11. Article absorbant (1) selon l'une quelconque des revendications 1 à 10, dans lequel un rapport entre la densité de polymère absorbant (P) de l'extension longitudinale (7F) de la seconde couche (7) et la densité de polymère absorbant (P) de la première couche (6), en termes de dernier/premier, est de 0,05 ou plus et de 0,5 ou moins.

12. Article absorbant (1) selon l'une quelconque des revendications 1 à 11, dans lequel l'article absorbant (1) est une couche jetable.
